Europäisches Patentamt

European Patent Office

Office européen des brevets

(19)

(11) Veröffentlichungsnummer: **0 305 756 B1**

(12) **EUROPÄISCHE PATENTSCHRIFT**

(45) Veröffentlichungstag der Patentschrift: **23.12.92**

(21) Anmeldenummer: **88112630.4**

(22) Anmeldetag: **03.08.88**

(51) Int. Cl.5: **A61K 9/70**, A61M 35/00, G01N 33/48

(54) **Vorrichtung zur Abgabe von Stoffen, Verfahren zu ihrer Herstellung sowie ihre Verwendung.**

(30) Priorität: **01.09.87 DE 3729165**
**23.12.87 DE 3743945**

(43) Veröffentlichungstag der Anmeldung:
**08.03.89 Patentblatt 89/10**

(45) Bekanntmachung des Hinweises auf die Patenterteilung:
**23.12.92 Patentblatt 92/52**

(84) Benannte Vertragsstaaten:
**AT BE CH DE ES FR GB GR IT LI LU NL SE**

(56) Entgegenhaltungen:
EP-A- 0 086 468     EP-A- 0 127 282
EP-A- 0 144 486     EP-A- 0 186 019
WO-A-88/01516       US-A- 4 515 909

(73) Patentinhaber: **LTS Lohmann Therapie-Systeme GmbH & Co. KG**
**Irlicherstrasse 55**
**W-5450 Neuwied 12(DE)**

(72) Erfinder: **Jaeger, Halvor**
**Brühlweg 9-11**
**W-7910 Neu-Ulm(DE)**
Erfinder: **Hoffmann, Hans-Rainer**
**Burghofstrasse 123**
**W-5450 Neuwied 22(DE)**
Erfinder: **Meconi, Reinhold**
**Alemannenstrasse 42**
**W-5451 Neuwied 11(DE)**
Erfinder: **Klein, Robert-Peter**
**Wikingerstrasse 3**
**W-5450 Neuwied 11(DE)**

(74) Vertreter: **Neidl-Stippler, Cornelia, Dr.**
**Rauchstrasse 2**
**W-8000 München 80(DE)**

Rank Xerox (UK) Business Services

## Beschreibung

Die Erfindung betrifft eine Vorrichtung zur Abgabe von Stoffen aus Haftschmelzklebern mit ungleichmäßiger oder gleichmäßiger Verteilung der Stoffe in den Haftschmelzklebern sowie ein Verfahren zu deren Herstellung und deren Verwendung.

Typische Repräsentanten derartiger Vorrichtungen sind wirkstoffhaltige Pflaster, Indikatorsysteme, Duftstoffe abgebende Vorrichtungen und dgl., wobei diese insbesondere im medizinischen Bereich für die gesteuerte oder ungesteuerte Abgabe von Stoffen häufig eingesetzt werden. Hierbei haben die gesteuerten Vorrichtungen in Form transdermal gesteuerter Systeme eine besondere Bedeutung erlangt. Für diese ist es auch bereits bekannt geworden, eine wirkstoffhaltige Schicht aus der Schmelze aufzutragen. Aus der EP - A - 0177893 ist ein nichtklebendes, aus der Schmelze aufgetragenes Celluloseethergel bekannt geworden, in dem Wirkstoffe verteilt werden können . Dieses Gel wird heiß verarbeitet und ist nichtklebend. Aus der DE-OS 32 22 800 ist ein transdermales System bekanntgeworden, bei dem in Mikrokapseln verpackter Wirkstoff in einem thermisch formbaren, klebenden Matrixmaterial vorliegt, das aus der Schmelze aufgetragen wird.

Aus der US-A-4515909 sind Artikel bekannt geworden, bei denen Polymere bei erhöhter Temperatur in festem Zustand mit Duftstoff imprägniert werden. Dort wird der Kunststoff nicht geschmolzen, sondern ein etwas erweichtes Polymeres mit dem Duftstoff unter erhöhten Temperaturen imprägniert.

Dieses Verfahren eignet sich nicht zur exakten Einbringung definierter Mengen in ein Polymeres.

Es wurde auch versucht, für temperaturempfindliche Wirkstoffe mit niedrigem Siedepunkt oder auch leichter Zersetzlichkeit nichtklebende wirkstoffhaltige Matrices bei Raumtemperatur herzustellen. Bspw. wird in der US-PS 4,379,454 (Campbell et al.) angegeben, eine mittels Geliermittel bei Raumtemperatur auf einen erwünschten Viskositätswert gebrachte Wirkstofflösung für die Wirkstoffschicht einzusetzen. Aus der US-PS 4,559,222 (Enscore et al.) ist bekannt, eine bei Raumtemperatur hergestellte Mischung von Mineralöl/Polyisobutylen und kolloidalem Siliciumdioxid als viskose Wirkstoffschicht für öllösliche Wirkstoffe einzusetzen, wobei diese Schichten auch haftklebend ausgebildet sein können. Die DE - OS - 32 22 800 (ALZA) beschreibt eine Wirkstoffschicht aus einer mittels eines rheologischen Mittels, wie Cellulose, einem Polysaccharid oder einer Siliciumverbindung angedickten Wirkstofflösung, die nichtklebend ist und sich für schnelle Wirkstoffabgabe eignet.

Aus der US-PS 3,923,939 ist bekannt geworden, Wirkstoffe, wie Tetracyclin, in einer Ethylen-Vinylacetat-Copolymerschicht durch Schmelzpressen zu formen. In der EP-A 86 468 wird ein orales Antidiabetes-Sulfonylharnstoffderivat in einer nichtklebenden Heißschmelzmasse mit einem Schmelzpunkt von 30 bis 90 °C in vorherbestimmten Dosen aus der Schmelze in Kapseln abgefüllt. In der US-A-3,957,966 ist beschrieben, daß Wirkstoffe in nichtklebenden Heißschmelzmassen verarbeitet werden können.

Aus der DE-A-30 07 363 ist bekannt geworden, eine haftklebende Mischung aus einem thermoplastischen Elastomeren, hier bevorzugt einem Blockpolymeren der allgemeinen Formel ABA einem klebrigmachenden Harz mit Öl oder höheren Fettsäuren und Wirkstoffen zur Herstellung eines einfachen transdermalen Systems einzusetzen. Die dort beschriebene Haftschmelzklebermischung eignet sich nur für relativ temperaturresistente Wirkstoffe, die Temperaturen von 120° C oder mehr vertragen.

In der US-PS 3,699,963 ist beschrieben, daß Oxytocin mit Haftkleber zur Herstellung eines transdermalen therapeutischen Systems vermischt und bei einer Temperatur oberhalb von 90 °C verformt werden kann. Die derart hergestellten transdermalen Systeme sind preiswert in der Herstellung und sichern über die ganzflächige Anklebung des Systems an der Haut eine konstante Wirkstoffübertragung.

Die Verfahren nach dem Stand der Technik zur Herstellung derartiger Systeme eignen sich nicht für transdermale Systeme, die temperaturempfindliche Stoffe, wie Scopolamin, beinhalten. Bisher wurden daher haftklebende Wirkstoffschichten mit temperaturempfindlichen Wirkstoffen bevorzugt aus der Lösung gebildet und das Lösemittel verdampft.

Die Verwendung von Lösemitteln bei der Herstellung wirkstoffhaltiger Kleberschichten ist aus mehreren Gründen nachteilig. Die Herstellung der Lösungen erfordert mindestens einen zusätzlichen aufwendigen Verfahrensschritt. Sie bedingt einen hohen technischen Aufwand und Kosten für die Handhabung der Lösemittel - außerdem müssen für medizinische Zwecke hochreine und damit teure Lösemittel eingesetzt werden, um für die Auflösung der Kleber bzw. deren Ausgangsmaterialien eine entsprechende Rückstandsfreiheit in der Vorrichtung sicherzustellen. Ein weiteres Problem besteht darin, Lösemittelfreiheit in der Vorrichtung zu erreichen, wofür teure Trocknungsstrecken und Absauganlagen erforderlich sind. Zusätzlich treten Kosten durch Wiedergewinnung oder Abscheidung der Lösemittel auf, um Umweltbelastung zu vermeiden. Daneben stellt die Brennbarkeit der meisten Lösemittel ein zusätzliches Riksiko dar. Außerdem sind die meisten organischen Lösemittel schädlich für den menschlichen Organismus, so daß aufwendige Schutzmaßnahmen für die im Betrieb tätigen Personen getroffen werden müssen. Es ist somit Aufgabe der Erfindung, die oben aufgeführten Nachteile der gattungsgemäßen Vorrichtungen und Verfahren nach dem Stand der Technik zu vermeiden.

Die Aufgabe wird erfindungsgemäß durch eine Vorrichtung zur Abgabe von Stoffen aus Haftschmelzklebern mit ungleichmäßiger oder gleichmäßiger Verteilung der Stoffe in den Haftschmelzklebern gelöst, bei der eine Haftschmelzkleber/Stoffmischung oder eine Lösung des Stoffes im Haftschmelzkleber vorliegt, wobei der Haftschmelzkleber eine Verarbeitungstemperatur zwischen 40 und 80 °C, bevorzugt zwischen 40 und 60 °C und ganz besonders bevorzugt zwischen 40 und 55 °C hat.

Hierdurch kann bei niedrigen Temperaturen ohne Lösemittel gearbeitet werden, wodurch eine erhebliche Ersparnis an Materialien, eine beschleunigte Herstellung der Vorrichtung ohne die zeitaufwendigen Trocknungsschritte sowie eine die Umwelt weniger belastende Herstellung von erfindungsgemäßen Vorrichtungen ermöglicht werden, was unter anderem zu einem erheblich kostengünstigeren und auch lösemittelfreien Produkt führt.

Die Vorrichtung kann dabei den/die abzugebenden Stoff(e) und Haftschmelzkleber als eine oder mehrere Schichten aufweisen. Bevorzugt ist der Haftschmelzkleber mit einem für den/die abzugebenden Stoff(e) durchlässigen oder undurchlässigen Träger ausgerüstet.

Der Haftschmelzkleber kann beispielsweise ein solcher sein, der auf Basis von Styrolisoprenstyrolblockpolymeren, Polyaprolactonen, Ethylen-Vinylacetat-Copolymeren, Polyurethan, Polyepoxiden, Polyisobuten, Polyvinylethern, ggf. unter Zusatz von Weichmachern, Tackifiern, Füllstoffen, Alterungsschutzmitteln und/oder Thixotropiermitteln hergestellt ist.

Ein erfindungsgemäßes Verfahren zur Herstellung einer erfindungsgemäßen Vorrichtung beinhaltet kontinuierliches oder diskontinuierliches Aufbringen abzugebenden Stoff enthaltenden geschmolzenen Haftschmelzklebers bei einer Temperatur des Haftschmelzklebers zwischen 40 und 80 °C, bevorzugt 40 bis 60 °C und besonders bevorzugt 40 bis 55 °C auf einen Träger und ggf. Anbringen des Schutzschichtmaterials.

Ein weiteres erfindungsgemäßes Verfahren beinhaltet kontinuierliches oder diskontinuierliches Aufbringen abzugeben den Stoff enthaltenden geschmolzenen Haftschmelzklebers bei einer Temperatur des Haftschmelzklebers zwischen 40 und 80 °C, bevorzugt 40 bis 60 °C und besonders bevorzugt 40 bis 55 °C auf ein Schutzschichtmaterial und ggf. Anbringen des Trägers.

Beim Einsatz leichtflüchtiger und/oder thermisch instabiler abzugebender Stoffe können die nachfolgend genannten besonderen Maßnahmen zur Verarbeitung angezeigt sein:

A. das Arbeiten bei möglichst tiefen Temperaturen

B. die Erhöhung des Außendrucks, um die Verdampfung herabzusetzen,

C. die Sättigung des Dampfraums über der Schmelze mit dem Dampfförmigen Stoff

D. Das Arbeiten mit entsprechend den Vorgaben kleinstmöglicher Menge an flüchtigem Stoff in der Schmelze.

Selbstverständlich sind diese Maßnahmen, wie bspw. das Arbeiten in einer gekapselten Anlage, durch die dem Fachmann durch den Einsatzzweck der herzustellenden Vorrichtung als auch die stofflichen Gegebenheiten bekannten Gesetzmäßigkeiten limitiert.

Die erfindungsgemäßen Vorrichtungen, insbesondere die transdermalen Systeme, lassen sich bspw. für die lokale oder systemische transdermale Wirkstoffverabreichung in der Human- oder Tiermedizin oder der Kosmetik, bevorzugt zur Abgabe von temperaturempfindlichen und/oder leichtflüchtigen Stoffen einsetzen.

Dabei wird hier unter Haftschmelzkleber jeder Haftklebstoff verstanden, der heiß hinreichend flüssig ist, um problemlos bei einer Temperatur oberhalb etwa 40 °C aufgetragen zu werden.

Als erfindungsgemäß einsetzbare Haftschmelzkleber können unter anderem solche eingesetzt werden, die dem Fachmann geläufig sind und wie sie u. a. in den DE - A - 15 94 268 (SUN OIL CO.), DE-A- 24 13 979 (E.I DU PONT DE NEMOURS)-,DE-A-24 35 863 (DYNAMIT NOBEL AG); DE - A - 28 00 302 (CIBA GEIGY); den EP - A - 104 005 (PERSONAL PRODUCTS CO), den JP 6104 2583 und JP 61 281 810, der EP - A - 131 460 (EXXON) und der EP -A - 234 856 (EXXON), der EP - A - 185 992 (EASTMAN KODAK) sowie der US-PS 36 99 963 und der US-PS 4,358,557 (EASTMAN KODAK) herleitbar sind, wobei zur Vermeidung von Wiederholungen auf diesen Stand der Technik ausdrücklich bezug genommen wird.

Dabei können als Grundpolymere bspw. Polyamide, Polyester, Polycaprolactame, Polycaprolacton, Ethylen-Vinylacetat-Copolymere (EVA), Ethylen-Ethylacrylat-Copolymere (EEA), Polyvinylether,Polyacrylatester, Polyvinylacetale, Polyvinylacetate, Styrol-Butadien-Blockpolymere, Isopren-Blockpolymere, Polyurethane, Ethylcellulose, Celluloseacetat-Butyrat, Synthesekautschuke (z B. Neopren-Kautschuk), Polyisobutylen, Butylgummi, Acrylnitril-Butadien-Mischpolymerisate, Epoxidharze, Melaminharze, Phenol-Formaldehyd-Harze und Resorcin-Formaldehyd-Harze verwendet werden, wobei auch unter anderem die nachfolgend genannten modifizierenden Harze eingesetzt werden können: hydriertes Kolophonium, polymerisiertes Kolophonium, dimerisierte Harzsäuren, disproportioniertes Kolophonium, Methylester von Kolophonium, Glycerinester von hydriertem Kolophonium, Methylester von hydriertem Kolophonium, Pentalester, Triethylenglykolester von hydriertem Kolophonium, Hydroabiethylalkohol und dessen Derivate, Glycerinester, Di-Triolester und Pentaester von Harzsäuren, Pentalester von polymerisiertem Kolophonium, Pentalester von dimerisiertem Kolophonium, Glycerinester von dimerisiertem Kolophonium, Ester von Maleinsäure- oder Phenolmodifiziertem Kolophonium, aromatische und aliphatische Kohlenwasserstoffharze, hydrierte Harze, Polyterpenharze modifizierte Terpenharze, Wachse, niedermolekulares Polyethylen und Polypropylen, Alkyl-Styrol-Copolymere. Diesen Harzen können ggf Weichmacher, wie z.B. Adipinsäureester, Phosphorsäureester, Phthalsäureester, Polyester, Fettsäureester, Citronensäureester oder Epoxidweichmacher zugesetzt werden. Außerdem können auch Stabilisatioren, wie Tocopherol, substituierte Phenole, Hydrochinone, Brenzkatechine, aromatische Amine, und ggf. auch noch Füllstoffe, wie z. B. Titandioxid, Magnesiumoxid, Zinkoxid und Siliciumdioxid zugemischt werden.

Die Bildung der Bestandteile der Vorrichtung, die Haftschmelzkleber mit einer Verarbeitungstemperatur zwischen 40 und 80 °C aufweisen, kann durch Extrusion, Gießen, Walzenauftrag, Rakelauftrag, Aufsprühen oder ein Druckverfahren erfolgen.

Ein Grenzwert für die Verarbeitbarkeit der Haftschmelzkleber bei vielen dieser Verfahren ist bei einer Viskosität im Bereich von etwa 80 000 Pa•s gegeben.

Falls die mit dem Kleber zu behandelnde Unterlage - eine Komponente der Vorrichtung - durch die Temperatur des heiß aufgetragenen Klebers beschädigt werden könnte - sei es durch Zersetzung, Reaktion oder partielles Schmelzen, kann eine gekühlte Unterlage eingesetzt werden. Die Kühlung kann durch an sich bekannte Verfahren erfolgen, wie durch Einbringung kalter inerter Gase oder das Kontaktieren mit einer Kühlfläche.

Der Haftschmelzkleber kann bspw. in Schichtform oder in einzelnen Bereichen entsprechend einem vorherbestimmten Muster auf die Schutzschicht oder das Abdeckmaterial aufgetragen werden.

Je nach Anwendungszweck - falls bspw. der abzugebende Stoff durch die Rückschicht abgegeben werden soll, wie es bspw. bei ätherischen Ölen, wie Duftstoffen sein kann, kann der Haftschmelzkleber mit einem für den/die abzugebenden Stoff(e) durchlässigen Träger ausgerüstet sein, während bei der Ausführungsform der Vorrichtung als transdermales System, bei dem der Stoff nur an die Haut abzugeben ist, eine für den abzugebenden Stoff undurchlässige Rückschicht bevorzugt ist.

Durch das erfindungsgemäße Verfahren kann nun die Verwen dung von lösemittelhaltigen haftklebenden Materialien bei der Verarbeitung von temperaturempfindlichen und leicht flüchtigen Stoffen umgangen werden, was zu erheblicher Erhöhung der Sicherheit der Herstellung, da nun sicher keine toxischen Lösemittelreste in der Arzneimitteldarreichungsform verbleiben, einem stark vereinfachten Verfahren der Auftragung und zu erheblicher Einsparung der Herstellungskosten führt. Dabei kann das Verfahren natürlich auch in vorteilhafter Weise für wenig temperaturempfindliche Stoffe eingesetzt werden, da sich auch dabei erhebliche Kostenersparnis erzielen läßt.

Unter der Bezeichnung "Stoffe" im Zusammenhang mit der vorliegenden Erfindung werden chemische Elemente, organische und anorganische Verbindungen, die aus den sie enthaltenden Bestandteilen einer gattungsgemäßen Vorrichtung herauswandern können und dadurch einen angestrebten Effekt hervorrufen, verstanden. Unter den Anwendungsgebieten der erfindungsgemäßen Vorrichtung ist die Human- und Tiermedizin von besonderer Bedeutung, wobei hier eine Ausgestaltung der Erfindung in Pflasterform besonders bevorzugt ist.

Typische, über erfindungsgemäß hergestellte Vorrichtungen verabreichbare Stoffe sind hierbei: Aceclidin, Amfetaminil, Amfetamin, Amylnitrit, Apophedrin, Atebrin, Alprostadil, Azulen, Arecolin, Anethol, Amylenhydrat, Acetylcholin, Acridin, Adenosintriphosphorsäure, L-Äpfelsäure, Alimemazin, Allithiamin, Allyl-Isothiocyanat, Aminoethanol, Apyzin, Apiol, Azatadin, Alprenolol, Äthinazon, Benzoylperoxid, Benzylalkohol, Bisabolol, Bisnorephedrin, Butacetoluid, Benactyzin, Campher, Colecalciferol, Chloralhydrat, Clemastin, Chlorobutanol, Capsaicin, Cyclopentamin, Clobutinol, Chamazulen, Dimethocain, Codein, Chlorpromazin, Chinin, Chlorthymol, Cyclophosphamid, Cinchocain, Chlorambuzil, Chlorphenesin, Diethylethan, Divinylethan, Dexchlorpheniramin, Dinoproston, Dixyrazin, Ephedrin, Ethosuximid, Enallylpropymal, Emylcamat, Erytroltetranitrat, Emetin, Enfluran, Eucalyptol, Etofenamat, Ethylmorphin, Fentanyl, Fluanison, Guajazulen, Halothan, Hyoscyamin, Histamin, Fencarbamid, Hydroxycain, Hexylresorcin, Isoaminilcitrat, Isosorbiddinitrat, Ibuprofen, Jod, Jodoform, Isoaminil, Lidocain, Lopirin, Levamisol, Methadon, Methyprylon, Methylphenidat, Mephenesin, Methylephedrin, Meclastin, Methopromazin, Mesuximid, Nicethamid, Norpseudoephedrin, Menthol, Methoxyfluran, Methylpentynol, Metixen, Misoprostol, Oxytetracain, Oxyprenolol, Oxyphenbutazon, Oxychinolin, Pinen, Prolintan, Procyclidin, Piperazin, Pivazid, Phensuximid, Procain, Phenindamin, Promethazin, Pentetrazol, Profenamin, Perazin, Phenol, Pethidin, Pilocarpin, Prenylamin, Phenoxybenzamin, Resochin, Scopolamin, Salicylsäureester, Spartein, Trichlorethylen, Timolol, Trifluoperazin, Tetracain, Trimipramin, Tranylcypromin, Trimethadion, Tybamat, Thymol, Thioridazin, Valproinsäure, Verapamil, sowie weitere, dem Fachmann geläufige, über die Haut, eingeschlossen Schleimhäute, aufnehmbare Wirkstoffe. Selbstverständlich ist diese Aufzählung nicht abschließend.

Typische Zusammensetzungen für einzusetzende Haftschmelzklebstoffe sind solche, die aus zwischen 10 und 180 Gew.%, bevorzugt 20 bis 80 Gew.% und besonders bevorzugt 20 bis 50 Gew.% Polymer, zwischen 1 und 80 Gew.%, bevorzugt 15 bis 60 Gew.% Weichmacher; zwischen 1 und 80 Gew.%, bevorzugt 15 bis 60 Gew.% Tackifier, ggf. 0.1 bis 5 Gew.% Alterungsschutzmittel und ggf.0 bis 70 Gew.% Füllstoffe hergestellt sind, wobei die Summe der Prozentsätze der Bestandteile stets 100 ist.

Bevorzugt ist, daß der Haftschmelzklebstoff zu 10 bis 50 Gew.% Styrol - Isopren - Styrol Synthesekautschuk, wie es bspw. im Handel unter der Bezeichnung CARIFLEX TR 1107 von der Fa. SHELL erhältlich ist; zwischen 10 und 80 Gew.% eines hydrierten Alkohols, wie er bspw. unter der Bezeichnung ABITOL von der Fa. HERCULES erhältlich ist: zwischen 10 und 80 Gew.% eines Kohlenwasserstoffharzes, bspw. des Harzes HERCURES C von der Fa. HERCULES, zwischen 1 und 40 Gew.% an Estern pflanzlicher Fettsäuren, bspw. MIGLYOL 812 von der Fa. DYNAMIT NOBEL und ggf. bis zu 5 Gew.% Alterungsschutzmittel, wie Hydrochinon etc. sowie bis zu 70 Gew.% Füllstoffe aufweist.

Bei einer weiteren bevorzugten Ausführungsform der Erfindung weist der Haftschmelzklebstoff zu 10 bis 50 Gew.% eines Polycaprolactons, bspw. CAPA 650 von der Fa.INTEROX; zwischen 10 und 80 Gew.% eines hydrierten Alkohols, bspw. ABITOL von der Fa. HERCULES zwischen 10 und 80 Gew.% eines Kohlenwasserstoffharzes, bspw. HERCURES C von der Fa. HERCULES, zwischen 1 und 40 Gew.% an Estern pflanzlicher Fettsäuren, wie MIGLYOL 812 von der Fa. DYNAMIT NOBEL und ggf. bis zu 5 Gew.% Alterungsschutzmittel, sowie bis zu 70 Gew.% Füllstoffe auf.

Es kann auch bevorzugt sein, daß der Haftschmelzklebstoff zu 10 bis 50 Gew.% Polyethylen-Vinylacetat, wie EVATANE 28-25 von der Fa.ATOCHEM, zwischen 10 und 80 Gew.% eines hydrierten Alkohols bspw. ABITOL von der Fa. HERCULES, zwischen 10 und 80 Gew.% eines Kohlenwasserstoffharzes, bsw. des Harzes HERCURES C von der Fa. HERCULES zwischen 1 und 40 Gew.% an Estern pflanzlicher Fettsäuren bspw. MIGLYOL 812 von der Fa. DYNAMIT NOBEL und ggf. bis zu 5 Gew.% Alterungs-schutzmittel, wie Hydrochinon etc. sowie bis zu 70 Gew.% Füllstoffe aufweist.

Ein geeigneter Haftschmelzklebstoff kann zu 10 bis 50 Gew.% Polyurethan; wie z.B. LUPHEN P 1110 der Fa. BASF zwischen 10 und 80 Gew.% eines hydrierten Alkohols, bspw. ABITOL von der Fa. HERCULES zwischen 10 und 80 Gew.% eines Kohlenwasserstoffharzes, bspw. des Harzes HERCURES C von der Fa.HERCULES zwischen 1 und 40 Gew.% an Estern pflanzlicher Fettsäuren bspw. MIGLYOL 812 von der Fa. DYNAMIT NOBEL und ggf. bis zu 5 Gew.% Alterungsschutzmittel, sowie bis zu 70 Gew.% Füllstoffe aufweisen.

Es ist auch möglich, daß der Haftschmelzklebstoff zu 10 bis 50 Gew.% Polyamid, wie z.B. EURELON 930 der Fa. SCHERING zwischen 10 und 80 Gew.% eines hydrierten Alkohols, bspw. ABITOL von der Fa. HERCULES zwischen 10 und 80 Gew.% eines Kohlenwasserstoffharzes, bspw. des Harzes HERCURES C von der Fa. HERCULES zwischen 1 und 40 Gew.% an Estern pflanzlicher Fettsäuren bspw. MIGLYOL 812 von der Fa. DYNAMIT NOBEL und ggf. bis zu 5 Gew.% Alterungsschutzmittel, sowie bis zu 70 Gew.% Füllstoffe aufweist.

Es kann auch ein Haftschmelzklebstoff mit 10 bis 50 Gew.% Epoxid, bspw. z.B. EUREPOX 7001 der Fa. SCHERING, zwischen 10 und 80 Gew.% eines hydrierten Alkohols, bspw. ABITOL von der Fa. HERCULES zwischen 10 und 80 Gew.% eines Kohlenwasserstoffharzes, bspw. des Harzes HERCURES C von der Fa.HERCULES zwischen 1 und 40 Gew.% an Estern pflanzlicher Fettsäuren bspw. MIGLYOL 812 von der Fa.DYNAMIT NOBEL und ggf. bis zu 5 Gew.% Alterungsschutzmittel, wie Hydrochinon etc. sowie bis zu 70 Gew.% Füllstoffe verwendet werden.

Ein weiterer für die Herstellung erfindungsgemäßer transdermaler Systeme verwendbarer Haftschmelzklebstoff weist zu 10 bis 50 Gew.% Polyisobuten mit zähklebriger kautschukartiger Konsistenz, wie z.B. OPPANOL B 50 der Fa. BASF, zwischen 10 und 80 Gew.% eines hydrierten Alkohols, bspw. ABITOL von der Fa. HERCULES, zwischen 10 und 80 Gew.% eines Kohlenwasserstoffharzes, bspw. des Harzes HERCURES C von der Fa. HERCULES, zwischen 1 und 40 Gew.% an Estern pflanzlicher Fettsäuren, bspw. MIGLYOL 812 von der Fa. DYNAMIT NOBEL und ggf. bis zu 5 Gew.% Alterungsschutzmittel sowie bis zu 70 Gew.% Füllstoffe auf.

Schließlich ist es bevorzugt, Haftschmelzklebstoffe auf Polyesterbasis, die bspw. zwischen 10 und 80 Gew.% eines hydrierten Alkohols bspw. ABITOL von der Fa. HERCULES, zwischen 10 und 80 Gew.% eines Kohlenwasserstoffharzes, bspw. des Harzes HERCURES C von der Fa.HERCULES

zwischen 1 und 40 Gew.% an Estern pflanzlicher Fettsäuren bspw. MIGLYOL 812 von der Fa. DYNAMIT NOBEL und ggf. bis zu 5 Gew.% Alterungs-schutzmittel, sowie bis zu 70 Gew.% Füllstoffe aufweisen, einzusetzen.

Erfindungsgemäße Vorrichtungen können ferner auch ein oder mehrere Stoffdepots, in dem/nen der Stoff mit gegenüber der wirkstoffaufweisenden Haftschmelzklebstoffschicht erhöhter Konzentration vorliegt, besitzen, wodurch höhere Dosen des Stoffes verarbeitet werden können und damit die Vorrichtung länger im Einsatz bleiben kann, bevor sie ausgewechselt werden muß. Typische Ausgestaltungen finden sich bspw. in der DE-OS 36 29 304.0. Bevorzugte Ausgestaltungen der Erfindung sind in den Unteransprüchen aufgeführt, auf die hiermit ausdrücklich bezug genommen wird.

Weitere Merkmale und Vorteile der Erfindung werden nach stehend anhand der begleitenden Zeichnung erläutert. Dabei zeigt:

Fig.1 einen schematisch dargestellten Schnitt durch die Schichten einer erfindungsgemäßen Vorrichtung mit einem Stoffdepot.

Fig.2 einen schematisch dargestellten Schnitt durch eine weitere erfindungsgemäße Vorrichtung mit Wirkstoffdepot, und

Fig.3 einen schematisch dargestellten Schnitt durch eine weitere Ausführungsform einer erfindungsgemäßen Vorrichtung ohne Stoffdepot.

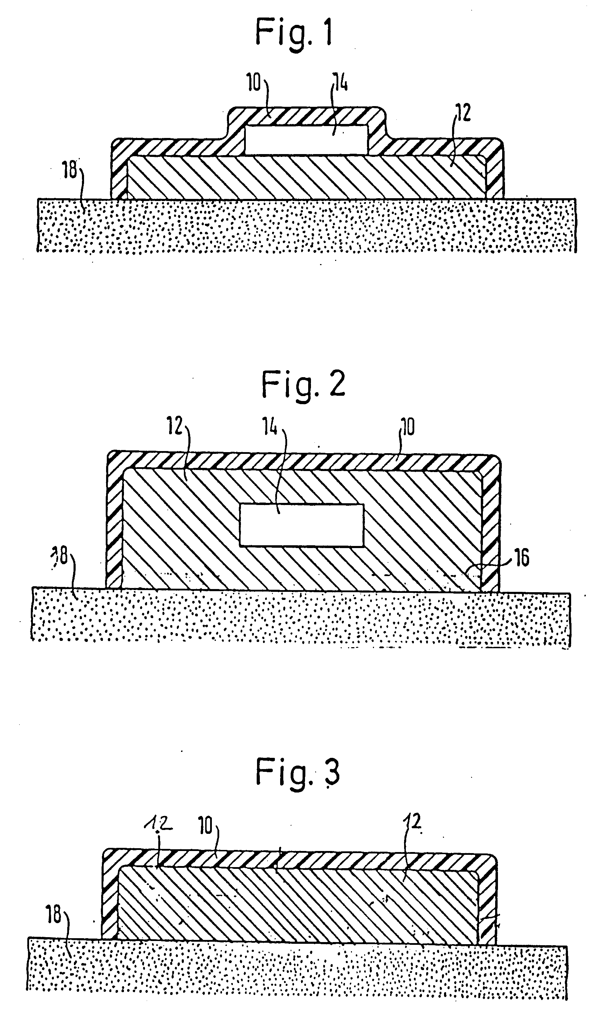

In Fig. 1 ist eine erfindungsgemäße Vorrichtung, die hier als pflasterartiges, wirkstoffhaltiges transdermales therapeutisches System ausgebildet ist, dargestellt. Sie besitzt eine Haftschmelzkleberschicht 12, ein Wirkstoffdepot 14, in dem der Wirkstoff zumindest eine höhere Konzentration als in der Haftschmelzkleberschicht 12 aufweist, sowie einen wirkstoff-undurchlässigen Träger 10, auf dem das Wirkstoffdepot 14 aufliegt, und ist auf der Haut 18 aufgeklebt. Es wandert nun kontinuierlich Wirkstoff mit einer vorherbestimmten Rate durch die Haut 18, wodurch der Wirkstoffgehalt in der Schicht 12 abnimmt. Die Wirkstoff abnahme wird durch Nachströmen von Wirkstoff aus dem Wirk stoffdepot 14 kompensiert, so daß über einen vorherbestimmbaren Zeitraum eine Gleichgewichtskonzentration des Wirkstoffes in der Haftschmelzkleberschicht 12 herrscht, die für die Abgabe einer konstanten Menge Wirkstoff an die Haut 18 sorgt.

In Fig. 2 ist eine weitere Ausführungsform einer erfindungsgemäßen Vorrichtung dargestellt, bei der ein Wirkstoffdepot 14 allseitig von der Haftschmelzkleberschicht 12 umgeben ist. Diese Ausführungsform eignet sich insbesondere dann, wenn eine große Kontaktfläche Wirkstoffdepot/Haftschmelzkleberschicht für eine schnelle Wirkstoffabgabe an die Haftschmelzkleberschicht erwünscht ist.

In Fig. 3 ist eine weitere einfache Ausführungsform einer erfindungsgemäßen Vorrichtung dargestellt, bei dem auf einem undurchlässigen Trägermaterial 10 eine wirkstoffhaltige Haftschmelzkleberschicht 12 derart aufgebracht ist, daß das Trägermaterial 10 die Schicht 12 dreiseitig überdeckt. Mit der freien Haftschmelzkleberfläche ist sie auf die Haut 18 aufgeklebt, so daß ein ganzflächiger Kontakt über die Applikationszeit gewährleistet ist und der Übergang des Wirkstoffes an die Haut stets über eine gleichbleibende Fläche mit einer gleichbleibenden Geschwindigkeit erfolgt.

Nachfolgend wird die erfindungsgemäß verbesserte Herstellung einer erfindungsgemäßen Vorrichtung beschrieben.
Zunächst wird eine Mischung der Komponenten des Haftschmelzklebers mit dem zu verabreichenden Stoff hergestellt. Diese Mischung wird auf Verarbeitungstemperatur gebracht und sodann aus der Schmelze auf ein Trägermaterial aufgebracht. Die weitere Verarbeitung, wie Aufbringen eines abhäsiv ausgerüsteten Schutzschichtmaterials, erfolgen in üblicher Weise.

Bezugszeichenliste

10 Träger
12 Haftkschmelzkleberschicht
14 Depot für abzugebenden Stoff
18 Haut

**Patentansprüche**

1. Vorrichtung zur Abgabe von Stoffen aus Haftschmelzklebern mit ungleichmäßiger oder gleichmäßiger Verteilung der Stoffe in den Haftschmelzklebern, dadurch gekennzeichnet, daß eine Haftschmelzkleber/Stoffmischung oder eine Lösung des Stoffes im Haftschmelzkleber vorliegt, wobei der Haftschmelzkleber eine Verarbeitungstemperatur zwischen 40 und 80 °C, bevorzugt zwischen 40 und 60 °C und besonders bevorzugt zwischen 40 und 55 °C besitzt.

2. Vorrichtung nach Anspruch 1, dadurch gekennzeichnet,daß der den/die abzugebenden Stoff(e) aufweisende Haftschmelzkleber als eine oder mehrere Schichten vorliegt.

3. Vorrichtung nach einem der Ansprüche 1 oder 2, dadurch gekennzeichnet, daß der Haftschmelzkleber mit einem für den/die abzugebenden Stoff(e) durchlässigen oder undurchlässigen Träger ausgerüstet ist.

4. Vorrichtung nach einem der vorangehenden Ansprüche,dadurch gekennzeichnet, daß der Haftschmelzkleber auf Basis von Styrol-Isopren-Styrol-Blockpolymeren, Polycaprolactonen, Ethylen-Vinylacetat-Copolymeren, Polyurethan, Polyepoxiden, Polyisobuten, Polyvinylethern, ggf.unter Zusatz von Weichmachern, Tackifiern, Füllstoffen, Alterungsschutzmitteln und/oder Thixotropiermitteln hergestellt ist.

5. Vorrichtung nach Anspruch 4, dadurch gekennzeichnet, daß der Haftschmelzkleber aus zwischen 10 und 80 Gew.%, bevorzugt 20 bis 80 Gew.% und besonders bevorzugt 20 bis 50 Gew.% Polymer, zwischen 1 und 80 Gew.%, bevorzugt 1 bis 60 Gew.% Weichmacher; zwischen 10 und 80 Gew.%, bevorzugt 15 bis 60 Gew.% Tackifier, ggf. 0.1 bis 5 Gew.% Alterungsschutzmittel und ggf. 0 bis 70 Gew.% Füllstoffe hergestellt ist, wobei die Summe der Prozentsätze der Bestandteile stets 100 ist.

6. Vorrichtung nach einem der vorangehenden Ansprüche, dadurch gekennzeichnet, daß sie eine ablösbare Schutzschicht aufweist.

7. Verfahren zur Herstellung einer Vorrichtung nach einem der vorangehenden Ansprüche, gekennzeichnet durch kontinuierliches oder diskontinuierliches Aufbringen einer geschmolzenen Mischung von Haftschmelzkleber und abzugebendem Stoff bei einer Temperatur der Mischung zwischen 40 und 80 °C, bevorzugt 40 bis 60 °C und besonders bevorzugt 45 bis 55 °C auf einen Träger und ggf. Anbringen eines Schutzschichtmaterials.

8. Verfahren zur Herstellung einer Vorrichtung nach einem der Ansprüche 1 bis 6, gekennzeichnet durch kontinuierliches oder diskontinuierliches Aufbringen einer geschmolzenen Mischung von Haftschmelzkleber und abzugebendem Stoff bei einer Temperatur der Mischung zwischen 40 und 80°C, bevorzugt 40 bis 60 °C und besonders bevorzugt 45 bis 55 °C auf ein Schutzschichtmaterial und ggf. Anbringen des Trägers.

9. Verfahren zur Herstellung einer Vorrichtung nach einem der Ansprüche 7 oder 8, dadurch gekennzeichnet, daß die Bildung der Bestandteile der Vorrichtung, die Haftschmelzkleber mit einer Verarbeitungstemperatur zwischen 40 und 80 °C aufweisen, durch Extrusion, Gießen, Walzenauftrag, Rakelauftrag, Aufsprühen oder ein Druckverfahren erfolgt.

10. Verwendung der Vorrichtung gemäß einem der Ansprüche 1 bis 6 in der Human- und Veterinärmedizin, Diagnostik oder der Kosmetik.

11. Verwendung der Vorrichtung gemäß Anspruch 10 zur Abgabe von temperaturempfindlichen und/oder leichtflüchtigen Stoffen.

**Claims**

1. Device for the release of substances from hot melt pressure sensitive adhesive having a non-uniform or uniform distribution of the substances in the hot melt pressure sensitive adhesive, characterised in that a mixture of hot melt pressure sensitive adhesive and substance or a solution of the substance in the hot melt pressure sensitive adhesive is present, the hot melt pressure sensitive adhesive having a processing temperature of between 40 and 80°C, preferably between 40 and 60°C and especially preferably between 40 and 55°C.

2. Device according to claim 1, characterised in that the hot melt pressure sensitive adhesive, having the substance(s) to be released, is present as one or more layers.

3. Device according to one of claims 1 or 2, characterised in that the hot melt pressure sensitive adhesive is provided with a carrier, which is permeable or impermeable to the substance(s) to be released.

4. Device according to one of the preceding claims, characterised in that the hot melt pressure sensitive adhesive is produced on the basis of styrene-isoprene-styrene block polymers, polycaprolactones, ethylene-vinylacetate copolymers, polyurethane, polyepoxides, polyisobutene, polyvinyl ethers, optionally with the addition of plasticizers, tackifiers, filler materials, anti-agers, and/or thixotropic agents.

5. Device according to claim 4, characterised in that the hot melt pressure sensitive adhesive is produced from between 10 and 80% by wt., preferably 20 to 80% by wt. and especially preferably 20 to 50% by wt., polymer, between 1 and 80% by wt., preferably 1 to 60% by wt. plasticizer; between 10 and 80% by wt. preferably 15 to 60% by wt., tackifier, optionally 0.1 to 5% by wt., anti-agers and optionally 0 to 70% by wt. filler materials, the sum of the percentages of the components always being 100.

6. Device according to one of the proceeding claims, characterised in that it has a removable protective layer.

7.  Method of producing a device according to one of the preceding claims, characterised by continuously or discontinuously applying a molten mixture of hot melt pressure sensitive adhesive and substance to be released at a temperature of the mixture of between 40 and 80°C, preferably 40 to 60°C and especially preferably 45 to 55°C, to a carrier and optionally applying a protective layer material.

8.  Method of producing a device according to one of claims 1 to 6, characterised by continuously or discontinuously applying a molten mixture of hot melt pressure sensitive adhesive and substance to be released at a temperature of the mixture of between 40 and 80°C, preferably 40 to 60°C and especially preferably 45 to 55°C, to a protective layer material and optionally the carrier.

9.  Method of producing a device according to one of claims 7 or 8, characterised in that the formation of the components of the device, which have hot melt pressure sensitive adhesive with a processing temperature of between 40 and 80°C, is effected by extrusion, moulding, roller application, knife coating, spraying or by a printing process.

10. Use of the device according to one claims 1 to 6 in the field of human and veterinary medicine, diagnostics or cosmetics.

11. Use of the device according to claim 10 for the release of temperature-sensitive and/or highly volatile substances.

**Revendications**

1.  Dispositif de diffusion de substances à partir de colles adhésives fusibles avec une distribution inégale ou uniforme des substances dans les colles adhésives fusibles, caractérisé en ce que l'on utilise un mélange de colle adhésive fusible et de substance ou une solution de la substance dans la colle adhésive fusible, la colle adhésive fusible ayant une température de traitement de 40 à 80°C, de préférence 40 à 60°C et tout particulièrement 40 à 55°C.

2.  Dispositif selon la revendication 1, caractérisé en ce que la colle adhésive fusible contenant la ou les substances à diffuser se présente sous la forme d'une ou plusieurs couches.

3.  Dispositif selon l'une quelconque des revendications 1 ou 2, caractérisé en ce que la colle adhésive fusible est dotée d'un support perméable ou imperméable à la ou aux substances à diffuser.

4.  Dispositif selon l'une quelconque des revendications précédentes, caractérisé en ce que la colle adhésive fusible est fabriquée sur base de copolymères-blocs de styrène/isoprène/styrène, de polyaprolactones, de copolymères d'éthylène/acétate de vinyle, de polyuréthane, de polyépoxydes, de polyisobutène, d'éthers polyvinyliques, éventuellement avec addition de plastifiants, d'agents adhésivants, de charges, d'agents anti-vieillissement et/ou d'agents thixotropes.

5.  Dispositif selon la revendication 4, caractérisé en ce que la colle adhésive fusible comprend 10 à 80 % en poids, de préférence 20 à 80 % en poids et mieux encore 20 à 50 % en poids de polymère, 1 à 80 % en poids, de préférence 1 à 60 % en poids de plastifiant, 10 à 80 % en poids, de préférence 15 à 60 % en poids d'agent adhésivant, éventuellement 0,1 à 5 % en poids d'agent anti-vieillissement et éventuellement 0 à 70 % en poids de charges, la somme des pourcentages des composants étant toujours de 100.

6.  Dispositif selon l'une quelconque des revendications précédentes, caractérisé en ce qu'il présente une couche protectrice enlevable.

7.  Procédé de fabrication d'un dispositif selon l'une quelconque des revendications précédentes, caractérisé par l'application continue ou discontinue d'un mélange fondu de colle adhésive fusible et de substances à diffuser à une température du mélange de 40 à 80°C, de préférence 40 à 60°C et mieux encore 45 à 55°C sur un support et éventuellement par l'application d'une matière de recouvrement protectrice.

8.  Procédé de fabrication d'un dispositif selon l'une quelconque des revendications 1 à 6, caractérisé par l'application continue ou discontinue d'un mélange fondu de colle adhésive fusible et de substances à diffuser à une température du mélange de 40 à 80°C, de préférence 40 à 60°C et mieux encore 45 à 55°C sur une matière de recouvrement protectrice et éventuellement par l'application du support.

9.  Procédé de fabrication selon l'une quelconque des revendications 7 ou 8, caractérisé en ce que la formation des composants du dispositif au cours de laquelle la colle adhésive fusible est soumise à une température de traitement de 40 à 80°C, se fait par extrusion, coulée, application au rouleau, application à la lame, pulvérisation ou par un procédé de compression.

10. Emploi du dispositif selon l'une quelconque des revendications 1 à 6 en médecine humaine et vétérinaire, en diagnostic ou en cosmétique.

11. Emploi du dispositif selon la revendication 10 pour la diffusion de substances thermosensibles et/ou volatiles.

# Fig. 1

# Fig. 2

# Fig. 3